# NEUE EUROPÄISCHE PATENTSCHRIFT

(11) **EP 3 954 312 B3**
(45) Veröffentlichungstag dieser Patentschrift: **26.06.2024**
(45) Hinweis auf die Patenterteilung: 20.09.2023
(21) Anmeldenummer: 21195035.7
(22) Anmeldetag: 30.05.2012
(51) Int. Cl.: A61B 17/70, A61B 17/86, A61B 90/00

(54) **CHIRURGISCHES INSTRUMTENT**
SURGICAL INSTRUMENT
INSTRUMENT CHIRURGICAL

(30) Priorität: 09.06.2011 DE 102011050996
(43) Veröffentlichungstag der Anmeldung: 16.02.2022
(62) Teilanmeldung aus: 12170044.7
(73) Patentinhaber: Combrowski, Zbiginiew, 78532 Tuttlingen (DE); Henninger, Alexander, 78570 Mühlheim (DE); Paroth, Christel, 78532 Tuttlingen (DE)
(72) Erfinder: Combrowski, Zbiginiew, 78532 Tuttlingen (DE); Henninger, Alexander, 78570 Mühlheim (DE); Paroth, Christel, 78532 Tuttlingen (DE)
(74) Vertreter: Westphal, Mussgnug & Partner Patentanwälte mbB

(56) Entgegenhaltungen:
- EP-A1- 1 774 919
- US-A1- 2008 045 953
- US-A1- 2008 082 103
- US-A1- 2009 299 415

## Beschreibung

### TECHNISCHES GEBIET

Die Erfindung betrifft ein Chirurgisches Instrument nach den Merkmalen des Oberbegriffs des Anspruchs 1.

### STAND DER TECHNIK

Die Indikationen für eine Spondylodese (Versteifung) der gesamten Wirbelsäule oder benachbarter Wirbel sind sehr vielschichtig. Stabilisierungen werden beispielsweise durchgeführt bei degenerativ veränderter Wirbelsäule, nach Tumorentfernungen, Infektionen oder Trauma.

In diesem Zusammenhang wird auf die US 2008/0082103 A1 hingewiesen, die ein System für das Einsetzen von Pedikelschrauben mit den Merkmalen des Oberbegriffs des Anspruchs 1 offenbart. Weiter wird auf die US 2009/0299415 A1 hingewiesen, welche eine Pedikelschraube mit einer angepassten Tulpe offenbart, wobei die Pedikelschraube schwenkbar in der Tulpe anordnebar ist. Daneben wird auf die US 2008/0045953 A1 hingewiesen, welche eine Pedikelschraube offenbart, welche über ein Stabelement, welches in derTulpe festlegbar ist, mit einer benachbarten Pedikelschraube mit Tulpe verbunden werden kann. Außerdem offenbartdie EP 1 774 919 A1 eine polyaxial schwenkbare Pedikelschraube.

ZurStabilisierung der Wirbelsäule kommen vielfach Kombinationen dorsaler Systeme (z.B. Stab-Pedikelschrauben-Systeme) in Kombination mit einem oder mehreren Cages zur ventralen Abstützung zum Einsatz.

Aus der Biomechanik ist bekannt, dass die Wirbelsäule als lasttragende Einheit mit der Statik eines Krans verglichen werden kann. Die vordere Säule, die aus Wirbelkörpern und Bandscheiben besteht, trägt ca. 80% der Last, die dorsalen Strukturen tragen ca. 20%. Daraus lässt sich ableiten, dass an der Lendenwirbelsäule der anteriore Anteil vorrangig Druckkräften, der dorsale Anteil vorrangig Zugkräften ausgesetzt ist. Zusätzlich wirken Scher-, Torsions- und Beugekräfte auf die beiden Säulen ein.

Während im dorsalen Bereich die obengenannten Schrauben/Stab-Systeme zum Einsatz kommen können, wird zur ventralen Abstützung ein Cage bzw. ein Implantat verwendet.

Ziel der Stabilisierung der Wirbelkörper ist eine schnelle Verknöcherung des Zwischenwirbelfaches, um eine dauerhafte Schmerzfreiheit des Patienten zu erreichen.

Beispiele für die Indikation einer Spondylodese sind: Instabile Wirbelfrakturen, degenerative Instabilitäten, Frakturen mit genügend anteriorer Abstützung, Dislokationen, Spinale Tumore (ohne anteriore Defekte), fehlgeschlagene vorgängige Fusion (Pseudoarthrose).

Die OP-Techniken finden je nach Indikation offen oder als minimal invasive Eingriffe statt. Unterschieden werden diese Verfahren generell in der Größe und Art des Zuganges bzw. der Zugänge.

Zugänge zur betroffenen Region können aus einer Kombination aus ventralem und dorsalem Zugang (an der Halswirbelsäule teilweise rein ventral) oder über einen dorsalen, dorso-lateralen oder lateralen Zugang erreicht werden.

In Abhängigkeit der vorgefundenen Strukturen und Größenverhältnisse wird ein Implantat in der richtigen Abmessung ausgewählt. In der Regel wird die Größe so bemessen, dass die Schraube bis ins vordere Drittel des Wirbelkörpers reicht.

Pedikelschrauben werden durch den Pedikel in den Wirbelkörper eingeschraubt. Zur sicheren Navigation, Führung sind manche Schrauben kanuliert und können dadurch über einen Führungsdraht eingebracht werden.

Manche besitzen zusätzliche Querbohrungen im Gewindeteil zum anschließenden Zementieren.

In den Kopf der Schraube, auch als "Tulpe" bezeichnet, wird ein Stab eingelegt, der zwei oder mehrere Pedikelschrauben miteinander verbindet. Die Tulpe kann starr oder in eine oder mehrere Richtungen beweglich sein, um das spätere Einbringen des Stabes zu erleichtern.

Nachdem der Stab auf beiden Seiten eingelegt ist wird mit unterschiedlichen Spreizsystemen, meist über die Pedikelschrauben die zu fixierenden Wirbelkörper, auseinandergedrückt um den optimalen Abstand der Wirbelkörper zu erreichen.

Dabei kann eine multidirektionale Beweglichkeit der Tulpe hinderlich sein, wenn es darum geht, Wirbelkörper aufzurichten oder ein physiologisches Alignment zu rekonstruieren (hauptsächlich in der Unfallchirurgie).

Minimal invasive Verfahren bedingen eine Verlängerung derTulpe um das extrakorporale, perkutane Einbringen des Stabes zu ermöglichen.

Je nach OP-Technik kommen von einem Hersteller verschiedene Schraubenformen und die dazugehörigen Instrumenten-Sets zum Einsatz.

Nachteiligerweise bestehen diese aus einer Vielfalt von Instrumenten, so dass nur erfahrene Operateure und OP-Assistenten dieses Instrumentarium ohne Produktmanager der Herstellfirmen anwenden können.

### AUFGABE DER ERFINDUNG

Aufgabe der Erfindung ist es ein verbessertes chirurgisches Instrument zur Verfügung zu stellen, das die o.g. Nachteile behebt.

### LÖSUNG DER AUFGABE

Zur Lösung der Aufgabe führen die Merkmale des Anspruchs 1. Vorteilhafte Weiterbildungen der Erfindung sind Gegenstand der abhängigen Ansprüche.

Ein chirurgisches Instrument umfasst eine Tulpe, wobei die Tulpe einen Druckring und/oder ein Führungselement zur Aufnahme einer Pedikelschraube aufweist. Bevorzugt ist der Druckring auswechselbaran der Tulpe angeordnet. In typischen Ausführungsbeispielen ist/sind das oder die Führungselemente einstückig an dem Druckring ausgeformt.

In typischen Ausführungsbeispielen ist/sind das oder die Führungselemente ein separates Bauteil. Vorteilhafter wird/werden ein oder mehrere Druckringe in Kombination mit einem oder mehreren Führungselementen, das/die ein separates Bauteil sind, verwendet.

Bevorzugt ist der Druckring und/oder das Führungselement jeweils einteilig ausgebildet. Damit ist gemeint, dass der Druckring als ein Bauteil ausgebildet ist und/oder auch das Führungselement als ein Bauteil ausgebildet ist.

In alternativen Ausführungsbeispielen ist der Druckring und/oder das Führungselement mehrteilig ausgebildet. Damit ist gemeint, dass der Druckring aus mehreren Einzelteilen zusammengesetzt ist und/oder auch das Führungselement aus mehreren Eintelteilen zusammengesetzt ist. Dadurch ergibt sich der Vorteil, dass die Führungselemente oder der Druckring durch Aussparungen/Löcher in der Tulpe gesteckt werden können. Dadurch kann der Druckring und/oder das Führungselement vorteilhaft auf einfache Art und Weise montiert und getauscht werden. Beispielsweise weist der Druckring einen Innen- und/oder einen Aussenring auf.

In typischen Ausführungsbeispielen ist der Druckring und/oder das Führungselement verdrehbar und/oder verschiebbar. Bevorzugt ist der Druckring so ausgebildet, dass durch eine Positionsänderung des Druckrings die Führungseigenschaften des Druckrings geändert werden. Besonders bevorzugt weist der Druckring ein oder mehrere Führungselemente auf, die in Abhängigkeit einer Position des Druckrings mit einem Schraubenkopf in Eingriff stehen können. Dadurch werden in Abhängigkeit einer Position des Druckrings unterschiedliche Bewegungen des Schraubenkopfes, bzw. der Schraube zulassen.

Zweckmässigerweise weist der Druckring und/oder das Führungselement einen Schaft auf. Bevorzugt ist der Schaft über eine Sollbruchstelle mit dem Druckring und/oder dem Führungselement verbunden, so dass der Schaft abbrechbar ist. Dadurch ergibt sich der Vorteil, dass der Druckring und/oder das Führungselement auf einfache Art und Weise verdrehet, verschoben und/oder gewechselt werden kann.

Zweckmässigerweise weist der Druckring ein erstes Führungselement auf, wobei das Führungselement eine monoaxiale Relativbewegung der Pedikelschraube zu der Tulpe zulässt.

Bevorzugt weist der Druckring ein zweites Führungselement auf, das eine polyaxiale Relativbewegung der Pedikelschraube zu der Tulpe zulässt.

In typischen Ausführungsbeispielen umfasst der Druckring ein drittes Führungselement, das die Tulpe starr zu der Pedikelschraube fixiert.

In typischen Ausführungsbeispielen ist der Druckring und/oder das Führungselement einstückig mit der Tulpe verbunden. Dadurch ergibt sich der Vorteil, dass keine Bauteile eingesetzt werden müssen.

Bevorzugt sind die Führungselement über eine Sollbruchstelle mit der Tulpe und/oder dem Druckring verbunden. Dadurch ergibt sich der Vorteil, dass nicht benötigte Führungselemente ausgebrochen werden können. Dadurch ergibt sich der Vorteil, dass sich ein Druckring und/oder eine Tulpe für starre, monoaxiale und polyaxiale Lagerung der Pedikelschraube eignet. Dazu können dann die nicht benötigten Führungselemente an den Sollbruchstellen abgetrennt werden.

Gemäß der Erfindung weist das chirurgische Instrument eine Tulpenverlängerung auf, geeignet zum Verbinden mit dem Verlängerungsschaft. Besonders bevorzugt ist das Design des Verlängerungschaftes, der Tulpenverlängerung und der Tulpe immer gleich. Dadurch ergibt sich der Vorteil, dass die Pedikelschraube und die Tulpe mit deren Verlängerungsschaft auf einfache Art und Weise ausgewechselt werden können. Dadurch ergibt sich der Vorteil, dass der Operateur immer mit seinem gewohnten Instrument, Verlängerungsschaft und/oder Griff arbeiten kann.

Gemäß der Erfindung ist die Tulpenverlängerung über eine Sollbruchstelle mit der Tulpe verbunden. Dadurch ergibt sich der Vorteil, dass die Tulpe über die Tulpenverlängerung auf einfache Art und Weise von dem Verlängerungsschaft getrennt werden kann. Denkbar ist es auch die Tulpe mit der Tulpenverlängerung und dem Verlängerungsschaft aus einem Stück auszubilden.

Weiterhin weist der Verlängerungsschaft zumindest teilweise in seinem vorderen Bereich ein Verlängerungswinde auf.

Gemäß der Erfindung umfasst die Tulpenverlängerung, zumindest in ihrem hinteren Bereich ein Verbindungsgewinde. Bevorzugt ist das Verbindungsgewinde geeignet, mit dem Verlängerungsgewinde des Verlängerungsschafts in Wirkverbindung zu treten. Dadurch ergibt sich der Vorteil, dass der Verlängerungsschaft und die Tulpenverlängerung auf einfache Art und Weise miteinander verbunden werden können. Zweckmässigerweise ist das Verbindungs- bzw. das Verlängerungsgewinde bevorzugt entgegen zu der Drehrichtung eines Gewindes der Pedikelschraube ausgebildet.

Bevorzugt sind die Tulpenverlängerung und der Verlängerungsschaft einstückig ausgebildet sind. Die Tulpenverlängerung ist über ein Sollbruchstelle verbunden.

Vorteilhafterweise sind die Tulpe, die Tulpenverlängerung und der Verlängerungsschaft einstückig ausgebildet. Bevorzugt sind die Tulpe, die Tulpenverlängerung und der Verlängerungsschaft über Sollbruchstellen verbunden.

In alternativen Bauteilen sind die Tulpe, die Tulpenverlängerung und der Verlängerungsschaft Einzelteile, die über Gewinde, Stecken, Rasten und/oder Kleben miteinander verbunden werden.

Im typischen Ausführungsbeispiel ist der Verlängerungsschaft hohl. Dadurch ergibt sich der Vorteil, dass sich der Verlängerungsschaft zum Einbringen des Stabes, eines Schraubendrehers, eines Ini, eines Führungsdrahtes und zum Einbringen von Zement geeignet ist.

Bevorzugt ist der Schraubendreher und/oder die Pedikelschraube kanuliert. Dadurch ergibt sich der Vorteil, dass sich die Bauteile zum Einbringen des Führungsdrahtes und/oder Zement eignen. Zusätzlich kann in der Kanulierung der Pedikelschraube ein z.B. Innengewinde oder eine kupplungsähnliche Vorrichtung angeordnet sein, das/die geeignet ist ein Instrument oder Kartusche zum Zementieren anzudocken.

Das Eindrehen der Pedikelschraube kann auch über den Verlängerungsschaft mit z. B. dem Gegenhalter geschehen , so könnte auf den Schraubendreher für die Pedikelschraube verzichtet werden. Hierzu muss die Pedikelschraube in z.B. der Tulpe nur geklemmt werden, das kann mit einem einfacheren, günstigeren Instrument wie dem kanulierten Schraubendreher geschehen.

Gemäß der Erfindung weist die Tulpenverlängerung ein Innengewinde auf.

In typischen Ausführungsbeispielen umfasst das chirurgische Instrument einen Ini. Bevorzugt weist der Ini einen grossen Verstellweg auf. Dadurch ergibt sich der Vorteil, dass eine Verstellen über nur einen Ini-Antrieb und/oder einen T-Griff ohne weitere Zusatzinstrumente möglich ist.

Zweckmässigerweise umfasstderVerlängerungsschaft und/oder die Tulpenverlängerung einen Aussenkonus. Bevorzugt umfasst das Abbrechwerkzeug einen Innenring. Besonders bevorzugt ist der Innenring als Konus ausgebildet. Dadurch ergibt sich der Vorteil, dass der Verlängerungsschaft auch für den Arbeitsschritt "Abbrechen" einsetzbar ist. Wird ein Abbrechwerkzeug auf den Verlängerungsschaft aufgesetzt und zum Beispiel über einen nicht dargestellten Hebel herunter gedrückt, bricht der Verlängerungsschaft an der Tulpe an der Sollbruchstelle nach innen ab. Das Abbrechen kann auch seitlich oder nach aussen erfolgen.

Bevorzugt umfasst das chirurgische Instrument eine Pedikelschraube, wobei die Pedikelschraube einen einzigen Schraubenkopf aufweist, welcher mit einem ersten Führungselement und/oder dem zweiten Führungselement und/oder dem dritten Führungselement der Druckringe in Wirkverbindung bringbar ist.

Dadurch ergibt sich der Vorteil, dass zum Auswählen, ob zwischen Tulpe und Pedikelschraube keine Bewegung, eine monoaxiale Bewegung oder polyaxiale Bewegung zugelassen werden soll, das gleiche Instrumentationsset verwendet werden kann und nur der Druckring entsprechend ausgewechselt werden muss. dass der Druckring auswechselbar an der Tulpe angeordnet ist. Bevorzugt ist die Pedikelschraube kanuliert. Besonders bevorzugt umfasst die Pedikelschraube eine selbstbohrenden Spitze, geeignet zum Eröffnen des Pedikels. Besonders bevorzugt weist die Pedikelschraube ein selbstschneidendes Gewinde auf. Noch bevorzugter weist die Pedikelschraube einen teilweise zylindrischen und oder teilweise konischen Gewindeteil auf.

Sodass sich mehrere Gewindedurchmesser ergeben können, die z.B. über konische Übergänge miteinander verbunden sind.

Die Erfindung findet besonders vorteilhaft darin Anwendung, dass alle Pedikelschrauben, einen charakteristischen multifunktionellen Verlängerungsschaft mit gleichem Design der Tulpe, Tulpenverlängerungen und Verlängerungsschäfte aufweisen.

Bevorzugt werden Pedikelschrauben in verschiedenen Durchmessern ca. 5 mm, 6 mm und 7 mm, in einer Länge von 30 mm bis 70 mm hergestellt. Zu den Pedikelschrauben können Tulpen in polyaxialer, monoaxialer oder starrer Ausführung kombiniert werden. Bevorzugt sind alle Pedikelschrauben kanuliert, selbstschneidend und/oder in den Durchmessern 6 mm und 7 mm zementierbar ausgebildet.

Bevorzugt dient der Verlängerungsschaft als Führung für den Stab und Stabeinbringen und hält zugleich den Verschluss (Ini) für den Stab vormontiert bereit.

Über den Verlängerungsschaft finden alle Arbeitsschritte wie Eindrehen der Pedikelschrauben, Messen der Stablänge, im Bedarfsfall zementieren, Einbringen des Stabes, Distraktion und Kompression, Aufrichten der Wirbelkörper zur Rekonstruktion des physiologischen Alignments und Stabfixierung mit dem Ini statt.

Sind alle Stäbe in der Tulpe fixiert, wird der Stabeinbringer vom Stab entkoppelt, jetzt kann der Verlängerungsschaftdurch die SnapOff-Technik, also die Tulpenverlängerung von der Tulpe mit einem Instrument gratfrei extrakorporal abgetrennt werden.

Alle Pedikelschrauben können bevorzugt mit integriertem Ini und Bohrdraht besonders bevorzugt einzeln und steril verpackt angeboten.

Gemäß einem Beispiel umfasst ein kleines, übersichtliches erfindungsgemässes Instrument-Set, das für alle OP-Techniken gleich ist, einen Bohrdraht, Messinstrumente für die Schraubenlänge über den Bohrdraht, ein Kombiinstrument Gewindebohrer/Pedikelöffner, einen Schraubendreher/einen Antriebe für den Ini, einen Schraubendreher/Antriebe für die Pedikelschraube, einen T-Griff mit Ratsche mit integriertem Drehmomentbegrenzer, ein Tulpen/Verlängerungsschaft Abbrechwerkzeug, einen Gegenhalter und/oder ein Instrument zur Distraktion und Kompression mit Stablängenmessung.

In typischen Ausführungsbeispielen ist die Sollbruchstelle als Ring, zweiteilig oder mehrteilig ausgebildet. Typischerweise ergibt sich die mehrteilig ausgebildete Sollbruchstelle, weil sie beispielsweise durch Öffnungen zum Stab Ein- bzw. Ausbringen unterbrochen ist. Eine mehrteilige Ausbildung der Sollbruchstelle kann auch dazu führen, dass die Sollbruchstelle leichter zu trennen ist.

Bevorzugt wird als Material für die Pedikelschraube, Druckring, Tulpe, Tulpenverlängerung und den Ini Titan Grade 5 verwendet.

Bevorzugtes Herstellungsmaterial für den Verlängerungsschaft ist Edelstahlrohr. Gemäß der Erfindung ist der Verlängerungsschaft formschlüssig über ein Linksgewinde mit der Tulpenverlängerung verschraubbar. Weitere Verbindungsarten sind Löten, Kleben, Schweissen und Stecken.

Durch die Verwendung des erfindungsgemässen chirurgischen Instruments ergeben sich für den Operateur wichtige Vorteile, es können vormontierte Implantate mit Ini verwendet werden, der Verlängerungsschaft ist multifunktionell verwendbar, da über diesen alle Arbeitsschritte durchgeführt werden, das System ist zementierbar für offene und minimalinvasive OP-Techniken. Es wird ein schlankes einheitliches Instrument für alle OP-Methoden zur Verfügung gestellt. Alle Produkte sehen gleich aus und sind gleich bedienbar für monoaxiale, polyaxiale und starre Pedikelschrauben. Dadurch bestehen wenige Fehlerquellen und auch ungeübte Operateure erreichen mit diesem chirurgischen Instrument eine steile Lernkurve.

Bevorzugt wird das chirurgische Instrument in sterilen Einzelverpackungen angeboten. Besonders bevorzugt ist das Gewinde der Pedikelschraube, so ausgeführt, dass sich ein Maximum an Kompression ergibt.

In typischen nicht näher dargestellten Ausführungsbeispielen umfasst die Tulpe ausgeformte Taschen bzw. der Schraubenkopf, so dass die Druckringe von aussen und innen umschaltbar bzw. verdrehbar, verschiebbar und steckbar sind, so dass sich mit einem Druckring vor dem Sterilverpacken, vor oder während der OP die gewünschte zulässige Relativbewegung zwischen Tulpe und Schraubenkopf einstellen lässt.

Als Führungselemente zwischen Druckring und Schraubenkopf sind alle möglichen Elemente, die die Freiheitsgrade für starr, monoaxial beweglich oder polyaxial beweglich bieten, zulässig.

Ein Druckring kann beispielsweise aus einer Kombination von zwei Druckringen bestehen, oder Druckring oder Druckringe mit einem anderen Teil, wobei der innere oder äussere Druckring im Bedarfsfall vor dem Sterilverpacken, vor oder während der OP getauscht oder gewählt werden kann, um eine andere Schraubenaxialität zu erreichen.

Die Führungselemente und die Druckringe können zum einfachen Wechseln, Verdrehen, Verschieben etc. auch mit einem abbrechbaren Schaft mit einer SnapOff Abbrechstelle wie die Tulpe ausgestattet sein.

### KURZE BESCHREIBUNG DER ZEICHNUNGEN

Nachfolgend wird die Erfindung anhand der beiliegenden Figuren kurz beschrieben, wobei die Figuren zeigen:
- Figur 1: zeigt eine schematische Darstellung eines Sets mit einem erfindungsgemässen chirurgischen Instrument in einer Implantationssituation;
- Figur 2: zeigt eine Schnittdarstellung eines erfindungsgemässen chirurgischen Instruments;
- Figur 3: zeigt eine Seitenansicht des erfindungsgemässen chirurgischen Instruments, bei dem das Instrument an der Sollbruchstelle getrennt ist;
- Figur 4: zeigt eine schematische Darstellung eines vergrösserten, geschnittenen Ausschnitts des erfindungsgemässen Instruments;
- Die Figuren 5 bis 7: zeigen unterschiedliche erfindungsgemässe Ausführungsbeispiele eines Druckrings für ein erfindungsgemässes chirurgischen Instrument;
- Figur 8: zeigt eine schematische Darstellung einer perspektivischen Ansicht einer Pedikelschraube für ein erfindungsgemässes chirurgisches Instrument;
- Figur 9: zeigt eine vergrösserte perspektivische Darstellung eines Pedikelschraubenkopfes;
- Figur 10: zeigt einen vergrösserten, geschnitten Ausschnitt eines erfindungsgemässen chirurgischen Instruments nach Fig. 1.

### AUSFÜHRUNGSBEISPIEL

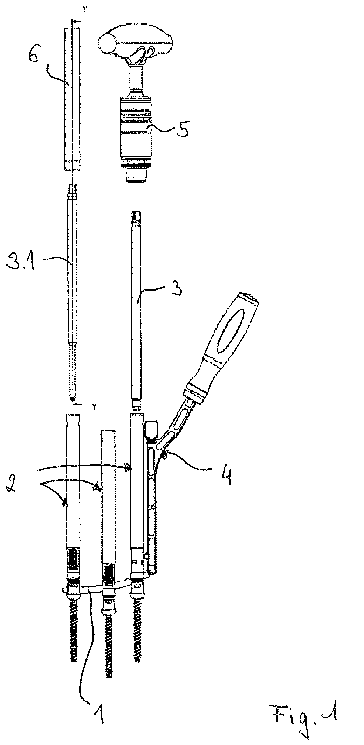

Figur 1 zeigt ein Stab-Pedikelschrauben-System zur Stabilisierung der Wirbelsäule. Dieses System umfasst einen Stab 1, drei Schraubeneinheiten 2 als erfindungsgemässes chirurgisches Instrument, einen Schraubendreher 3 für das Ini, eine Spannzange 4, einen T-Griff 5 mit Drehmomentbegrenzung, und eine nur teilweise dargestellte Abbrechhülse 6.

In Figur 1 ist auch ein Schraubendreher 3.1 für eine Pedikelschraube dargestellt.

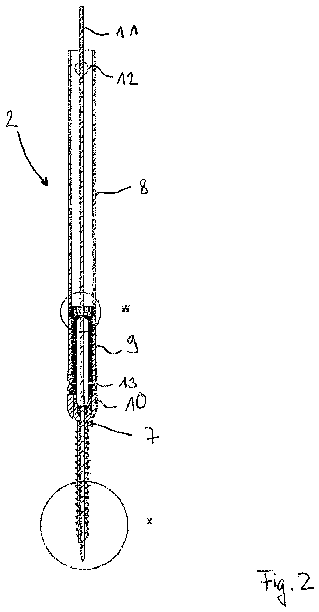

Figur 2 zeigt eine vergrösserte Schnittdarstellung der Schraubeneinheit 2. Die Schraubeneinheit 2 umfasst eine Pedikelschraube 7 und einen Verlängerungsschaft 8, eine Tulpenverlängerung 9 und eine Tulpe 10.

Der Verlängerungsschaft 8, die Tulpenverlängerung 9, die Tulpe 10 und die Pedikelschraube 7 sind kanuliert ausgebildet. In die Bauteile ist ein Führungsdraht 11 eingebracht. In einem oberen Bereich umfasst der Verlängerungsschaft 8 eine Öffnung 12, insbesondere geeignet zum Gegenhalten.

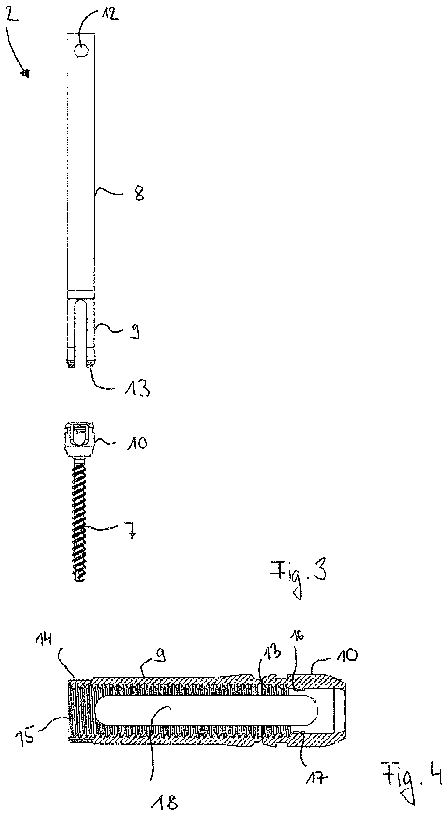

Die Tulpenverlängerung 9 und die Tulpe 10 sind über eine Sollbruchstelle 13 verbunden. Die Figur 3 zeigt die Schraubeneinheit 2, bei der Tulpenverlängerung 9 und die Tulpe 10 an der Sollbruchstelle 13 getrennt sind. Figur 4 zeigt eine vergrösserte Schnittdarstellung der Tulpenverlängerung 9 mit der Tulpe 10, die an der Sollbruchstelle 13 verbunden sind. Die Tulpenverlängerung 9 umfasst an einer Aussenseite an einem hinteren Bereich ein Verbindungsgewinde 14. Des Weiteren umfasst die Tulpenverlängerung 9 an ihrer Innenseite ein Innengewinde 15. Das Verbindungsgewinde 14 ist ein Linksgewinde. Das Innengewinde 15 ist ein Rechtsgewinde

Das Innengewinde 15 reicht bis in einen oberen Bereich der Innenseite der Tulpe 10.

Die Tulpe 10 weist an jeder Innenseite, in etwa auf mittlerer Höhe zwei Haltenasen 16 und 17 auf. In die Tulpenverlängerung 9 und die Tulpenverlängerung 10 ist ein Langloch 18 eingebracht, geeignet zur Aufnahme des Stabs 1.

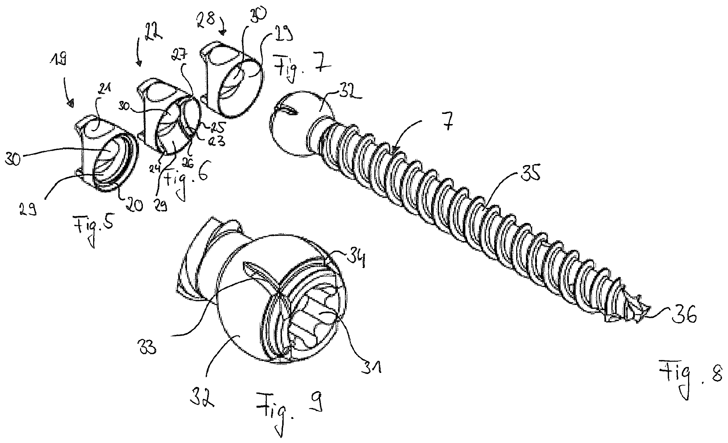

Figur 5 zeigt ein erstes Ausführungsbeispiel eines Druckrings 18. Der Druckring 19 weist eine halbschalenförmige Innenseite mit einer Öffnung 20 auf. Der Druckring 18 eignet sich zum starren Halten einer der Pedikelschraube 7. Dazu weist der Druckring 18 als Führungselement an seiner Innenseite eine umlaufende Nut 20 auf. Zum Festlegen des Druckrings 19 in der Tulpe 10 umfasst der Druckring 18 eine Vertiefung 21, in die die Haltenase 16 der Tulpe 10 wie in Figur 4 dargestellt, greifen kann. Gegenüberliegend zur Vertiefung 21 umfasst der Druckring 19 eine weitere, nicht dargestellt Vertiefung, die mit der Haltenase 17 in Wirkverbindung bringbar ist.

Figur 6 zeigt ein weiteres Ausführungsbeispiel eines Druckrings 22, geeignet zum monoaxialen Halten der Pedikelschraube 7. Der Druckring 22 ist im Wesentlichen analog zu dem Druckring 19 ausgebildet. Der Druckring 19 weist eine halbschalenförmige Innenseite mit einer Öffnung 20 auf. Der Druckring 22 unterscheidet sich von dem Druckring 19

in der Ausbildung des Führungselements. Als Führungselement weist der Druckring 22 einen ersten Steg 23 und einen zweiten Steg 24 auf. Die Stege 23 und 24 sind im Wesentlichen symmetrisch zueinander angeordnet. Der Steg 23 weist eine halbkreisbogenförmige Form auf. Der Steg 23 ist so an einer Innenseite des Druckrings angebracht, dass die Enden des Halbkreisbogens 26 und 27 an einer Kante 25 des Druckrings enden. Dabei handelt es sich bei der Kante 25 um die Kante, die der Pedikelschraube 7, bei montierter Schraubeneinheit 2 zugewandt ist.

Figur 7 zeigt ein weiteres Ausführungsbeispiel eines Druckrings 28. Der Druckring 28 ist im Wesentlichen analog zu den Druckringen der vorhergehenden Ausführungsbeispiele ausgebildet und unterscheidet sich in seinem Führungselement. Der Druckring 28 lässt eine polyaxiale Relativbewegung der Pedikelschraube 7 zu der Tulpe 10 zu. Dazu ist eine Innenseite 29 des Druckrings 28 als Halbschale 29 ausgebildet. In die Halbschale 29 ist eine nach oben gerichtete Öffnung 30 eingebracht.

Die Öffnung 30 in der Halbschale 29 dient der Bedingung eines Innenmehrkant 31 in einen Schraubenkopf 32 der Pedikelschraube 7, wie in Figur 9 dargestellt.

Wie in der vergrösserten Darstellung in Figur 9 des Schraubenkopfs 32 erkennbar, weist auch der Schraubenkopf 32 Führungselemente auf, die mit den Druckringen 19, 22 und 28 zusammenwirken.

Für alle Druckringe 19, 22 und 28 kann eine Pedikelschraube 7 mit einem Schraubenkopf 32 mit der gleichen Ausbildung eingesetzt werden. Der Schraubenkopf 32 ist kugelförmig ausgebildet, so dass er mit der halbschalenförmigen Innenfläche 29 der Druckringe 19, 22 oder 28 nach Art eines Kugelgelenks zusammenwirken kann.

Des Weiteren umfasst der Schraubenkopf 32 zwei Rillen 33 und 34. Die Rillen 33 und 34 sind als Vertiefungen an dem Schraubenkopf 32 ausgeformt. Die Rillen 33 und 34 sind im Wesentlichen symmetrisch zueinander ausgebildet. Die Rillen 33 und 34 verlaufen als Halbkreisbogen in seitlichen Bereichen des kugelförmigen Schraubenkopfes 32. Dabei sind die Rillen 33 und 34 so angeordnet, dass sie auf den Stege 23 und 24 des Druckrings 22 laufen und dadurch eine monoaxiale Bewegung zugelassen wird.

Die Pedikelschraube 7 umfasst an ihrem Pedikelschraubenschaft 35 ein Gewinde. Das Gewinde ist bevorzugt zweigängig, selbstbohrend und/oder selbstschneidend ausgebildet.

Insbesondere eine Spitze 36 des Pedikelschraubenschaftes 35 ist selbstschneidend und selbstbohrend ausgebildet. Des Weiteren ist der Pedikelschraubenschaft 35 bevorzugt konisch ausgebildet, so dass der Pedikelschraubenschaft 35 in einem vorderen Bereich der Spitze 36 einen geringeren Durchmesser als in einem hinteren Bereich, nahe des Schraubenkopfes 32, aufweist.

Bevorzugt ist die Pedikelschraube 7 kanüliert ausgebildet, wie beispielsweise in Figur 2 erkennbar. Dadurch ergibt sich der Vorteil, dass durch die Pedikelschraube 7 beispielsweise der Führungsdraht 11 eingebracht werden kann, und sich die Pedikelschraube 7 auch zum Einbringen von Zement eignet. Vorzugsweise umfasst die Pedikelschraube 7 in ihrem Pedikelschraubenschaft 35 durchgängige Bohrungen, durch die der in die Kanüle der Pedikelschraube 7 eingebrachte Zement nach aussen in den Wirbelkörper tritt und sich mit diesem verbinden kann.

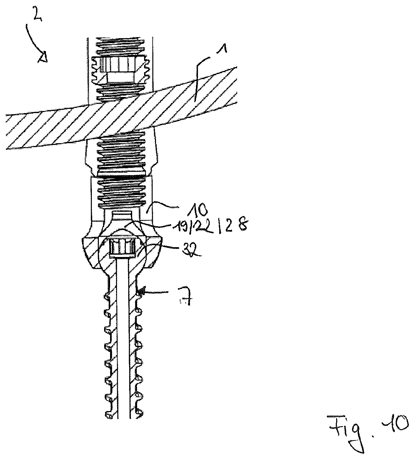

Die Figur 10 zeigt eine geschnitten und vergrösserte Darstellung der Schraubeneinheit 2 mit einem Teil des Stabes 1 nach der Figur 1. Es ist erkennbar, dass die Pedikelschraube 7 mit dem Pedikelschraubenkopf 32 in der Tulpe 10 aufgenommen ist. In die Tulpe 10 ist einer der Druckringe 19, 22 oder 28 eingesetzt und lässt die gewünschte Bewegung der Pedikelschraube 7 zu.

Die Funktionsweise der vorliegenden Erfindung ist folgende: Die Schraubenelemente 2 können je nach Indikation offen oder minimalinvasiv eingesetzt werden. Nach Bestimmung der Implantatdimension wird, bevorzugt unter Röntgenansicht mittels einer Jamshidi Nadel oder des Führungsdrahts 11 die korrekte Lage für die Pedikelschraube 7 ermittelt.

Anschließend werden der Führungsdraht 11 und/oder ein Portal eingesetzt. Zur Kontrolle der Pedikelschraubenlänge können mit weiteren nicht dargestellten Messinstrumenten über den Bohrdraht 11 die Schraubenlänge bestimmt bzw. verglichen werden.

Das Eröffnen des Pedikels und das Gewindeschneiden geschehen bevorzugt direkt mit dem selbstschneidenden Gewinde der Pedikelschraube 7 am Pedikelschraubenschaft 35. Die kanulierte Pedikelschraube 7 besitzt in dem Schraubenkopf 32 den Innenmehrkant 31, bevorzugt torxähnlich und wird sicher über den Führungsdraht 11 durch den Pedikel in den Wirbelkörper geführt und eingeschraubt.

Bei spröden, harten Knochen oder Glasknochenkrankheit sollte das Eröffnen des Pedikels und das Gewindeschneiden mit einem Kombiinstrument vorgenommen werden. Bevorzugt werden unterschiedliche Ausführungen eines Gewindebohrers eingesetzt.

Bei zu geringer Kompression oder osteoporösen Wirbelkörpern kann durch die kanulierte Pedikelschraube 7 nach dem Eindrehen Zement eingebracht werden.

Beim perkutanen Einführen des Stabes 1, wird der Stab 1 von oben durch den Verlängerungsschaft 8 eingebracht und von einer Seite durch das Langloch 18 in der Tulpenverlängerung 9 bzw. der Tulpe 10 wieder ausgebracht und durch den Wirbelkörper geführt. Hierdurch können zwei oder mehrere Wirbelkörper mit einem Stab 1 bis zu einer Länge von ca. 120 mm miteinander verbunden werden.

Nun erfolgt über den Verlängerungsschaft 8 extrakorporal die notwendige Distraktion oder Kompression, sowie ein Aufrichten der Wirbelkörper, um ein physiologisches Alignment zu rekonstruieren.

Die Distraktion oder Kompression wird über ein entlang des Verlängerungsschaftes 8 verstellbares Instrument erreicht. Mit diesem wird auch eine Länge des Stabes 1 bestimmt.

Ein notwendiges Aufrichten von Wirbelkörpern geschieht bevorzugt nur über den vormontierten Ini mit grossem Verstellweg und nur mit einem Ini-Antrieb 3 und dem T-Griff 5 ohne weitere Zusatzinstrumente.

Sind alle Stäbe in den Tulpen 10 der Schraubeneinheiten 2 fixiert und auf Drehmoment angezogen, kann der Verlängerungsschaft 8 an der Sollbruchstelle 13, die sogenannte SnapOff-Verbindung mit einem nicht dargestellten Instrument gratfrei abgetrennt werden. Alternativ wird dazu die Abbrechhülse 6 eingesetzt.

In weiteren Beispielen wird extrakorporal ein Tulpen-Verlängerungsschaft-Abbrechwerkzeug eingesetzt.

Zum Anziehen des Ini auf Drehmoment kann dem Verlängerungsschaft 8 extrakorporal ein nicht dargestellter Gegenhalter aufgesetzt werden. Dazu weist der Verlängerungsschaft 8 die Öffnung 12 auf.

Der Verlängerungsschaft 8 besitzt einen Aussenkonus, oberhalb der Sollbruchstelle 13 an der Tulpenverlängerung 9 und das Abbrechwerkzeug besitzt einen Innenring, der bevorzugt konusförmig ausgebildet ist. Wird das Abbrechwerkzeug auf den Verlängerungsschaft 8 aufgesetzt und zum Beispiel über einen nicht dargestellten Hebel herunter gedrückt, bricht der Verlängerungsschaft 8 an der Tulpe 10 an der Sollbruchstelle 13 nach innen ab. Das Abbrechen der Tulpe 10 kann aber auch nach aussen oder seitlich geschehen. Dazu kann ein Instrument, das an der Sollbruchstelle 13 teilweise an- oder abschneidet, verwendet werden.

Sobald die Stäbe 1 auf Drehmoment angezogen sind, kann auch der Stabeinbringer vom Stab entkoppelt und aus dem Körper entnommen werden.

### Bezugszeichenliste

| | | | | | |
|---|---|---|---|---|---|
| 1 | Stab | 34 | Rille | 67 | |
| 2 | Schraubeneinheit | 35 | Pedikelschraubenschaft | 68 | |
| 3 | Schraubendreher | 36 | | 69 | |
| 4 | Spannzange | 37 | | 70 | |
| 5 | T-Griff | 38 | | 71 | |
| 6 | Abbrechhülse | 39 | | 72 | |
| 7 | Pedikelschraube | 40 | | 73 | |
| 8 | Verlängerungsschaft | 41 | | 74 | |
| 9 | Tulpenverlängerung | 42 | | 75 | |
| 10 | Tulpe | 43 | | 76 | |
| 11 | Führungsdraht | 44 | | 77 | |
| 12 | Öffnung | 45 | | 78 | |
| 13 | Sollbruchstelle | 46 | | 79 | |
| 14 | Verbindungsgewinde | 47 | | | |
| 15 | Innengewinde | 48 | | | |
| 16 | Haltenase | 49 | | | |
| 17 | Haltenase | 50 | | | |
| 18 | Langloch | 51 | | | |
| 19 | Druckring | 52 | | | |
| 20 | Nut | 53 | | | |
| 21 | Vertiefung | 54 | | | |
| 22 | Druckring | 55 | | | |
| 23 | Erster Steg | 56 | | | |
| 24 | Zweiter Steg | 57 | | | |
| 25 | Kante | 58 | | | |
| 26 | Bogenende | 59 | | | |
| 27 | Bogenende | 60 | | | |
| 28 | Druckring | 61 | | | |
| 29 | Halbschale | 62 | | | |
| 30 | Öffnung | 63 | | | |
| 31 | Innenmehrkant | 64 | | | |
| 32 | Schraubenkopf | 65 | | | |
| 33 | Rille | 66 | | | |

## Patentansprüche

1. Chirurgisches Instrument (2) mit einer Tulpe (10), einer Tulpenverlängerung (9) und einem Verlängerungsschaft (8),
wobei die Tulpe (10) einen Druckring (19, 22, 28) und/oder ein Führungselement zur Aufnahme einer Pedikelschraube (7) aufweist,
wobei die Tulpenverlängerung (9) über eine Sollbruchstelle (13) mit der Tulpe (10) verbunden ist,
wobei die Tulpenverlängerung (9) an einer Aussenseite an einem hinteren Bereich ein Verbindungsgewinde (14) aufweist und an ihrer Innenseite ein Innengewinde (15) umfasst,
und wobei der Verlängerungsschaft (8) zumindest teilweise in seinem vorderen Bereich ein Verlängerungsgewinde aufweist,
wobei das Verbindungsgewinde (14) geeignet ist, mit dem Verlängerungsgewinde in Wirkverbindung zu treten,
**dadurch gekennzeichnet, dass** das Verbindungsgewinde (14) ein Linksgewinde und das Innengewinde (15) ein Rechtsgewinde ist.

## Claims

1. Surgical instrument (2) comprising a tulip (10), a tulip extension (9) and an extension shaft (8),
wherein the tulip (10) comprises a thrust ring (19, 22, 28) and/or a guide element for receiving a pedicle screw (7), wherein the tulip extension (9) is connected to the tulip (10) via a predetermined breaking point (13),
wherein the tulip extension (9) comprises a connecting thread (14) on an outer side at a rear region and comprises an internal thread (15) on its inner side,
and wherein the extension shaft (8) comprises an extension thread at least partially in its front region,
wherein the connecting thread (14) is adapted to come into operative connection with the extension thread,
**characterised in that**
the connecting thread (14) is a left-hand thread and the internal thread (15) is a right-hand thread.

## Revendications

1. Instrument chirurgical (2) comprenant une tulipe (10), une rallonge de tulipe (9) ainsi qu'une tige de rallonge (8),
- la tulipe (9) a un anneau de pression (19, 22, 28) et/ou un élément de guidage pour recevoir une vis pédiculaire (7),
- la rallonge de tulipe (9) étant reliée à la tulipe (10) par un point de rupture de consigne (13),
- la rallonge de tulipe (9) ayant un filetage de liaison (14) sur le côté extérieur de la zone arrière et un filetage intérieur (15) sur son côté intérieur, et
- la tige de rallonge (8) comporte au moins partiellement dans sa zone avant un filetage de rallonge,
- le filetage de liaison (14) convenant pour coopérer avec le filetage de rallonge,
instrument chirurgical **caractérisé en ce que**
le filetage de liaison (14) est un filetage à gauche et le filetage intérieur (15) un filetage à droite.
